# EUROPEAN PATENT APPLICATION

(11) **EP 1 393 638 A1**
(43) Date of publication of application: **03.03.2004**
(21) Application number: 02730315.5
(22) Date of filing: 13.05.2002
(51) Int. Cl.: A23L 3/3409, A23L 3/3445, A23B 7/152, A23B 7/144

(54) **SULPHUR DIOXIDE GENERATOR FOR FRESH FRUIT PRESERVATION AND PREPARATION METHOD THEREOF**

(30) Priority: 17.05.2001 CL 11382001
(71) Applicant: Quimetal Industrial S.A., Maipu, Santiago (CL)
(72) Inventor: QUINTANAR SANTIBANEZ, Carmina, Aida, 2507 Maipu, Santiago (CL); GARCIA CASTRO, Gaston, Yanko, 7465 Casa E. La Reina, Santiago (CL)
(74) Representative: SUGRANES - VERDONCES - FERREGÜELA
(86) International application number: PCT/ES2002/000224
(87) International publication number: WO 2002/091861

(57) **Abstract**

This invention corresponds to a device that delivers sulfur dioxide (SO2) in a controlled manner after getting in touch the active ingredient of the device, sodium metabisulfite, with humidity. The most important characteristic of the invention is that emission control of the gas is obtained with one or several homogeneous layers of the active ingredient at different concentrations allowing, if required, for a multilayer generator system with fast and slow emission of SO2 making use additionally of physical barriers specially designed for controlling the flux of water and SO2 through them. The invention also discloses the methods of obtaining the SO2 generator device.

## Description

This invention belongs to the generation of sulfur dioxide (SO2) for using in fresh fruit conservation, specifically for table grapes in order to avoid damages by fungi, bacteria or other microorganisms during storage or transportation.

The SO2 is generated in a "controlled" way in a homogeneous layer of the active ingredient from sodium metabisulfite reacting with environmental humidity.

### Background of the invention

SO2 generators have being used for a long time for fruit conservation in good conditions during their transportation and storage in refrigerated containers. When the fruit is packaged a SO2 generator is put inside of the box with fruit to protect it from fungi and bacteria attack. During the time needed for the fruit to get low temperature, it is exposed to microorganisms attack wherein high concentrations of SO2 are needed. However once the fruit is at storage temperature, around 0 °C, low levels of SO2 are required. In this last step amounts of SO2 must be low to avoid fruit damages due to the gas effect but high enough to control micro-organism that trigger attack en these conditions. Several patents exist for SO2 generators using as a source of SO2 sodium metabisulfite, or sodium bisulphate with organic acids all of them as solid material (US. Patents: 3,559,562 of Carlson and Black, 1971; 6,046,243 of Wellinghoff et al., 2000; and Chilean Patent 38,597 of Clemes, 1993).

The system mentioned above are based on the generation of SO2 after the reaction of sodium metabisulfite, heterogeneous solid, with water (humidity) or with an anion of a generator and a hydronium ion. The characteristic of this kind of systems is the provision of SO2, which is not totally controllable therefore exposing the fruit to damages for SO2 excess or deficit. Furthermore if the SO2 action is required for a long term (months), normally at least two phases of these materials are used distributed in higher amounts within a system known as a two-steps one.

The known systems of one- or two-steps consist of dispersing the solid sodium metabisulfite into a laminant substance such as paraffin wax. This provoke several inconveniences such as a non uniform exposition area if the size of the solid is not uniform. If the solids are different in size, reactivity of the generator varies with the thickness making the SO2 delivery uncontrolled.

A detailed analysis of the above systems allows to classify them within three categories:
1. SO2 generator systems in which solid sodium metabisulfite is introduced inside of small pockets formed by continuos strips in such a way that each pocket is isolated from the next one by isolation of their four sides. This system consists of paper generators with the active ingredients loosed inside the pockets.
2. SO2 generator systems with the active ingredient dispersed over a binder and then fixed over a plastic material or paper as a support. These systems were initially developed by Carlson and then by Clemes (*supra*).
3. SO2 generator systems based on an acidic precursor acting over an anion of the SO2 generator (Wellinghoff et al.). These systems use as emission controller, layers or barriers regulating water diffusion for interaction with the acid delivered and diffusion of the hydronium ion toward the anion generator of the SO2.

Each one of these systems has its own limitations. So, the first type of generators which uses paper pockets for the active ingredient doesn't allow a controlled SO2 emission since the sodium metabisulfite is in a solid state concentrated and cumulated inside the pockets according to gravity forces only and not uniformly distributed inside the pockets. Also the amount of active ingredient inside each cell may be variable, regarding the amount and the size of the active particles. Another disadvantage of the paper generators is found when this paper absorbs water causing a concentrated and local emission of SO2 which normally gives place to deficiencies in SO2 distribution inside the storage box.

In the second type of systems the active ingredient is dispersed into the binder with a distribution which can be heterogeneous and of variable granulometry and therefore SO2 delivery is not totally controlled. This is expressed in the initial emissions, called fast step, since there is a high concentration of SO2 therefore damaging the fruit. Control of SO2 delivery can only be done based on the physical barrier for the input of humidity and the output of SO2. Consequently further emission, slow delivery step, has a low SO2 emission level due to the blockage caused by the physical barrier. This phenomenon produces a deficient control of bacteria and fungi.
The barriers used in the previous systems belong to barriers for general purposes, that means not for specific control of SO2 emission, being therefore not a completely manageable barrier for SO2 generation.

The third type of SO2 generators delivers SO2 after reaction of an acidic precursor and the salt or anion of the gas generator. Here hydronium ion formation can be controlled through the use of a physical barrier limiting water diffusion through the layer of the acidic precursor.

Controlled delivery of SO2 is therefore a main problem for the efficiency of the generators. The present invention overcome this problem using a multilayer system with different and homogeneous concentrations of active ingredient. This is obtained with the use of an active ingredient into a homogeneous layer and not a randomly dispersed active ingredient. In this way a control of the emissions by the active ingredient and the disposition of the layers into a multilayer system with physical barriers specially designed is obtained for controlling input of water vapour and output of SO2.

### Disclosure of the invention

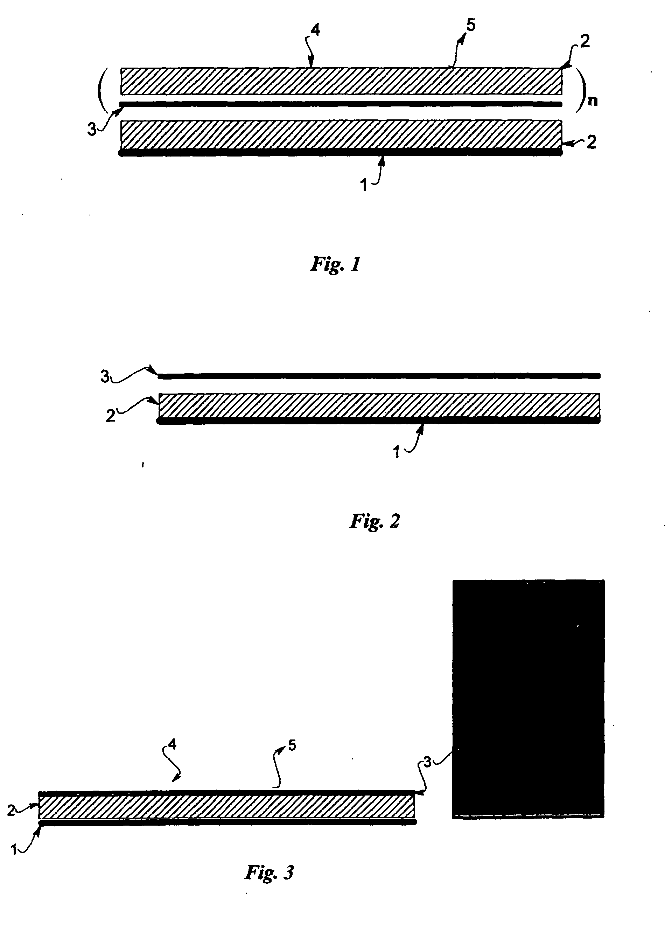

The invention is constituted by a device (Figure 1) which delivers SO2 (5) into a controlled manner from the homogeneous active ingredient (2) which contains sodium metabisulfite after reaction with humidity (4). The generated SO2 acts as a bactericide and fungicide of fruits, mainly table grapes. The device consists of a support (1) over which homogeneous layers of active ingredient are deposited as monolayers (Figure 2) or multilayers (Figure 1) which can be covered by a layer of physical barrier (3) which controls the input of water vapour and the output of SO2.

The invention also discloses the methods of preparation of the layers with active ingredients and also for the physical barrier layers forming the SO2 generator device.

As mentioned before, state of the art concerning manufacture of generators is based mainly in an eventual control of SO2 delivery by a physical barrier not specially designed for this purpose.

The actual invention instead make use of the control of the amount of active ingredient, sodium metabisulfite, which is homogeneously dissolved, and which is then mixed with the binder forming a uniform mixture for being deposited as a layer. This system allows for a fine chemical control of the active ingredient by the amount used and also through the formation of layers of different concentrations of active ingredient and with variable and controlled thickness producing a multilayer system. Use of a physical barrier furthermore allows to get a complete and practical control of SO2 generation over time.

The physical barrier of this invention is specifically designed for the purposes of controlling SO2 delivering and input of humidity based on the use of variable compositions of polymer with variable and controlled thickness.

This approach overcomes the problems and limitations known from the literature related to controlled SO2 delivery regarding the amount and variation of SO2 over time.

For obtaining the homogeneous layer of active ingredient an aqueous solution of the active ingredient, sodium metabisulfite or its precursor, sodium bisulphite, is mixed with the binder which is an aqueous emulsion of a polymer such as poly(vinyl acetate), poly(vinyl acrylate), polyethylene vinylacetate or with aqueous emulsion of resin such as acrylic styrene or polyester resins or with aqueous emulsions of waxes such as paraffin, polyethylene, polypropylene, o crystalline paraffin waxes. The concentration of the active ingredient is between 5 g/L - 560 g/L. Additionally, the binder is also a mixture of adhesive and resin in ratios between 10:90 w/w up to 90 to 10 w/w respectively. As a binder can also be used a mixture of adhesive and wax in ratios from 5:95 w/w to 95:5 w/w respectively. Another binary mixture, also used as binder, contains wax and resin in ratio from 40:60 w/w to 60:40 w/w respectively. Another type of binder uses a mixture of the three types of components, an adhesive, a resin, and a wax in ratios from 10:10:80 w/w to 70:1:29 w/w respectively.

By using any one of the options of the binders mentioned above and the aqueous solution of the active ingredient, a uniform mixture is obtained which is applied as a layer over the support which can be a sheet or a mesh made out of paper polyethylene with crown treatment of high, medium or low density, polypropylene with crown treatment of high, medium or low density, polystyrene, polyamide, polyester, polyurethane, PVC of food grade, or their mixtures.

Thickness of the layer with homogeneous active ingredient is regulated by the separation between the pervading cylinder over the flat surface of the support, or with several pervading cylinders with thickness controller between 5 µ to 1000 µ. This layer is submitted then to a drying step into an oven at 30-70 °C. Deposition of the layer over the support can be continuously (Figure 3) or discontinuously made with regular separations (Figure 4) or discontinuously with uneven separations (Figure 5).

The layer acting as a physical barrier is made out of aqueous emulsion of adhesives such as poly(vinyl acetate)s, poly(vinyl acrylate), ethylenevinylacrylate, ethylenevinylacetate, or with aqueous emulsion of resins such as acrylic, styrenic, polyesters or with aqueous emulsion such as paraffin polyethylene, polypropylene, microcrystalline paraffin waxes.

Additionally, the physical barrier is made out of a mixture of adhesive and resin in ratios between 20:80 w/w to 80:20 w/w respectively. A mixture of adhesive and wax is also used in ratios from 20:80 w/w up to 80:20 w/w respectively. Another mixture used for preparation of the physical barrier is a mixture of wax and resin in ratios from 10:90 w/w up to 90:10 w/w respectively.

Another mixture also uses the three components, an adhesive, a resin and a wax with ratios from 10:70:20 w/w up to 20:10:70 w/w respectively. Additionally, each component can be used as a bulk alone, heated and melted and deposited over the homogeneous layer of active ingredient. The thickness of the physical layer is regulated in the same way as the homogeneous layer of the active ingredient and varies in the range of 5 µ up to 500 µ.

The layer of the physical barrier **(3)**, of the Figure 1 and 2, is deposited over the homogeneous layer of the active ingredient **(2)**, acting by controlling the delivery of SO2 (5) from the homogenous layer of active ingredient **(2)** as a low and constant emission. Permeability to water vapour **(4)** and to the SO2 **(5)** is controlled by the thickness and the proportion of the constituents forming this physical barrier.

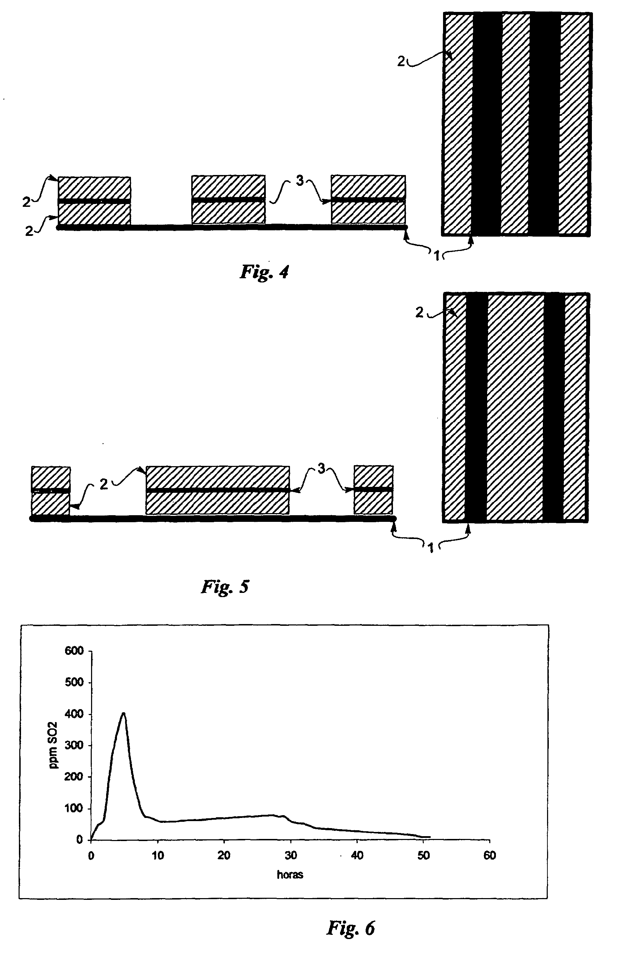

Multilayer device (Figure 1) is produced by consecutive applications of homogeneous layer of active ingredient and physical barrier alternatively or continuously, with a maximum of 20 layers. According to the specific requirements of use of SO2 generator device regarding levels of SO2 and time of emission, it may have one or more homogeneous layers of active ingredient as well as one or more physical barriers. In such a way a specific SO2 generator device is obtained which may has just one fast step of emission with short period of duration from 0 to 15 hr; another device may be one with a single slow step emission for a longer period of duration from 10 hr up; or one device containing both steps or phases together. These times are determined at room temperature.
General behaviour for SO2 delivery from a device with two-steps emission can be observed in Figure 6, obtained by periodic measurements with an SO2 detection equipment at room temperature. This Figure shows that SO2 emission levels of this invention are below 600 ppm of SO2 level. During the fast step I, deliveries of SO2 are higher than in step II, and duration of the slow step is larger than the fast step.

A correlation factor for the device between the experiment carried out at room temperature and the ones carried out at the storage temperature has been experimentally determined obtaining higher values for this last temperature, multiplying the factor for the time at room temperature. The factor is between 5 to 15 according to the device used. So if at room temperature SO2 duration delivery is 5 hours, at storage temperature duration delivery will be of 25 to 75 hours.

Typical embodiment of this invention for the preparation of a SO2 generator is as follows:

### Example

A SO2 generator device that delivers SO2 is manufactured according to the following procedure:

The homogeneous level of active ingredient is prepared from a mixture of 60 g of an emulsion of polyethylene wax, 145 mL of an aqueous solution of sodium metabisulfite at a concentration of 20 g/L, and 220 g of ethylene vinyl acetate adhesive. The obtained homogeneous mixture is applied as a layer with a thickness of 300 microns over a supporting base of a polypropylene sheet which is then submitted to a drying into an oven at 40 °C for 2 hr obtaining the homogeneous layer of active ingredient. Next, over the homogeneous layer of active ingredient a new layer of physical barrier is applied; this new layer is made from an adhesive of ethylene vinyl acetate and an emulsion of paraffin wax in a ratio of 30:70 w/w respectively, with a thickness of 200 microns. Then this double layer device is dried in an oven at 40 °C for 40 min.

Over the physical barrier an other layer of active ingredient is applied similarly to the one previously described , but with a thickness of 50 microns and again it is submitted to a new drying step.

### Description of the drawings

**Figure 1.** Transversal view of the SO2 generator as a multilayer system where n is the repeating unity.
**Figure 2.** Transversal view of the SO2 generator as a homogeneous monolayer of active ingredient, containing optionally a layer as a physical barrier.
**Figure 3.** Top view of the SO2 generator (right side) and a transversal view (left side), for a continuous deposition of the multilayers over the support.
**Figure 4.** Top view of the SO2 generator (right side) and a transversal view (left side), for a discontinuous deposition of the multilayers over the support.
**Figure 5.** Top view of the SO2 generator (right side) and a transversal view (left side), for a discontinuous irregular deposition of the multilayers over the support.
**Figure 6.** Graphic representation of a SO2 emission curve for a SO2 generator device having two phases, I and II, according to the invention.

## Claims

1. A SO2 generator device, for fruit conservation, wherein the SO2 generator device is a SO2 generator of controlled SO2 delivery, having a multilayer form with one, two, or more steps of emission of SO2, comprising a fast phase for short SO2 emission periods, and a slow one for larger periods.

2. A SO2 generator device, according to claim 1, wherein said generator is constituted by many layers over a support material where some layers are homogeneous layers of active ingredient and others are constituted of a physical barrier without any active ingredient.

3. A SO2 generator device, according to claim 1, wherein the SO2 is generated in the homogeneous phase of active ingredient starting from the reaction of the active ingredient, sodium metabisulphite, with water vapour.

4. A SO2 generator device, according to claim 1, wherein the SO2 emission is controlled by: i) the amount of active ingredient present in each layer, ii) the thickness of the homogeneous layer of active ingredient, iii) the composition and thickness of the physical barrier.

5. A SO2 generator device, according to claim 4, wherein the amount of active ingredient which is present in each of the homogeneous phases of active ingredient is from about 0.1 mg/cm² to 11 mg/cm².

6. A SO2 generator device, according to claim 4, wherein the thickness of each of the homogeneous phases of active ingredient varies between 5 µm and 600 µm.

7. A SO2 generator device, according to claim 4, wherein the thickness of the physical barrier varies between 5 µ and 500 µ, preferably between 20 µ and 400 µ.

8. A SO2 generator device, according to claim 2, wherein the support can be either a sheet or a mesh of: paper, polyethylene with a crown treatment of high, medium or low density, polypropylene with a crown treatment of high, medium or low density, polystyrene, polyamide, polyester, polyurethane, PVC of food grade, or mixtures of them.

9. A SO2 generator device, according to claim 2, wherein the superficial distribution of the multilayer over the support can be in a continuous way, regularly spaced, or discontinuously spaced.

10. A SO2 generator device, according to claim 9, wherein the superficial distribution of one or some layers within the multilayer over the support can be in a continuous way, regularly spaced, or discontinuously spaced.

11. A SO2 generator device, according to claim 2, wherein said generator comprises at least one homogeneous layer of active ingredient.

12. A SO2 generator device, according to claim 2, wherein said generator comprises two layers of active ingredient, one corresponding to the fast phase of SO2 generation, and the other to the slow phase, they being separated by a physical barrier.

13. A SO2 generator device, according to claim 2, wherein the homogeneous layers of active ingredient are prepared from a mixture of a binder with a solution containing the active ingredient or its precursor.

14. A SO2 generator device, according to claim 1, wherein an emission phase can be constituted by more than one homogeneous layer of active ingredient.

15. A SO2 generator device, according to claim 13, wherein the binder is an adhesive of the group of poly(vinyl acetate), poly(vinyl acrylate), ethylene vinyl acrylate, ethylene vinyl acetate or mixtures of them.

16. A SO2 generator device, according to claim 13, wherein the binder is a wax of the group of paraffin, polyethylene, polypropylene, microcrystalline paraffin or a mixture of them.

17. A SO2 generator device, according to claim 13, wherein the binder is a resin of the type acrylic, styrene, polyester, or a mixture of them.

18. A SO2 generator device, according to claim 13, wherein the binder is a mixture of an adhesive and a resin, in ratios from 10:90 w/w to 90:10 w/w. preferably 40:60 w/w to 60:40 w/w respectively.

19. A SO2 generator device, according to claim 13, wherein the binder is a mixture of an adhesive and a wax, in ratios from 5:95 w/w to 95:5 w/w preferably 5:95 w/w to 75:25 w/w respectively.

20. A SO2 generator device, according to claim 13, wherein the binder is a mixture of a wax and a resin, in ratios from 40:60 w/w to 60:40 w/w respectively.

21. A SO2 generator device, according to claim 13, wherein the binder is a mixture of an adhesive, a resin and a wax, in ratios from 10:10:80 w/w to 70:1:29 w/w respectively.

22. A SO2 generator device, according to claim 2, wherein at least one of the layers of the multilayer device corresponds to a physical barrier for controlling the input of water vapour and the output of SO2.

23. A SO2 generator device, according to claim 22, wherein the physical barrier is made with a resin of the type acrylic, styrene, polyester, or mixtures of them.

24. A SO2 generator device, according to claim 22, wherein the physical barrier is made with a wax of the type paraffin, polyethylene, polypropylene, microcrystalline paraffin, or a mixture of them.

25. A SO2 generator device, according to claim 22, wherein the physical barrier is made with an adhesive of the type poly(vinyl acetate), poly(vinyl acrylate), ethylene vinyl acrylate, ethylene vinyl acetate, or mixtures of them.

26. A SO2 generator device, according to claim 22, wherein the physical barrier is made with a mixture of an adhesive and a resin, in ratios from 20:80 w/w to 80:20 w/w preferably from 40:60 w/w to 60:40 w/w respectively.

27. A SO2 generator device, according to claim 22, wherein the physical barrier is made with a mixture of an adhesive and a wax, in ratios from 20:80 w/w to 80:20 w/w preferably from 40:60 w/w to 60:40 w/w respectively.

28. A SO2 generator device, according to claim 22, wherein the physical barrier is made with a mixture of a resin and a wax, in ratios from 10:90 w/w to 90:10 w/w preferably from 30:70 w/w to 70:30 w/w respectively.

29. A SO2 generator device, according to claim 22, wherein the physical barrier is made with a mixture of an adhesive, a resin, and a wax in ratios from 10:70:20 w/w to 20:10:70 w/w respectively.

30. A SO2 generator device, according to claim 2, wherein the multilayer comprises alternatively, a homogeneous layer of active ingredient and a physical barrier, this configuration being repeated for about 2 and 10 times.

31. A SO2 generator device, according to claim 2, wherein the multilayer consist of 20 layers as a maximum.

32. A SO2 generator device, according to claim 1, wherein the emission levels within a box of a volume similar to the volume of a fruit commercial box, preferably grapes, are under 600 ppm.

33. A SO2 generator device, according to claim 32, wherein the SO2 emission level in the fast phase is lower than 600 ppm.

34. A SO2 generator device, according to claim 32, wherein the SO2 emission level in the slow phase is lower than 500 ppm.

35. A SO2 generator device, according to claim 1, wherein the fast phase corresponds to a time of SO2 emission lower than or equal to 10 h at room temperature.

36. A SO2 generator device, according to claim 1, wherein the time of SO2 emission is controlled with the number of layers, the design of the active ingredient layer and the physical barrier, achieving, for a two phases system, that SO2 emission be kept for at least 3 months in storage conditions.

37. A method for preparing a SO2 generator device, according to claims 1 to 36, wherein the homogeneous layers of active ingredient correspond to a uniform mixture of an aqueous emulsion of a binder and a solution of the active ingredient or its precursor.

38. A method for preparing a SO2 generator device, according to claim 13, wherein the solution containing the active ingredient is made out of water and sodium metabisulphite, at a concentration from 5 g/L to 560 g/L.

39. A method for preparing a SO2 generator device, according to claim 13, wherein the solution containing the active ingredient precursor is made out of water and sodium bisulphite, at a concentration from 5 g/L to 560 g/L.

40. A method for preparing a SO2 generator device, according to claim 9, wherein the layers are formed with a pervading cylinder over a flat surface of the support, or with multiple pervaded cylinders that are thickness regulable to forming the layers.

41. A method for preparing a SO2 generator device, according to claims 22 to 29, wherein the physical barrier is prepared from an aqueous emulsion of the constituents or the component in bulk if required.
